(19)

**Europäisches Patentamt**

**European Patent Office**

**Office européen des brevets**

(11)  **EP 3 600 049 B1**

(12)  **EUROPEAN PATENT SPECIFICATION**

(45) Date of publication and mention
of the grant of the patent:
**25.11.2020   Bulletin 2020/48**

(21) Application number: **19712032.2**

(22) Date of filing: **20.02.2019**

(51) Int Cl.:
*A61B 6/00* *(2006.01)*        *G01V 5/00* *(2006.01)*
*G09B 23/28* *(2006.01)*

(86) International application number:
**PCT/IB2019/051391**

(87) International publication number:
**WO 2019/162860 (29.08.2019 Gazette 2019/35)**

(54)  **METHOD FOR CALIBRATING MAMMOGRAPHY EQUIPMENT, METHOD FOR GENERATING MAMMOGRAPHY IMAGES, AND MAMMOGRAPHY EQUIPMENT**

VERFAHREN ZUR KALIBRIERUNG VON MAMMOGRAFIEAUSRÜSTUNG, VERFAHREN ZUR ERZEUGUNG VON MAMMOGRAFIEBILDERN UND MAMMOGRAFIEAUSRÜSTUNG

PROCÉDÉ D'ÉTALONNAGE D'ÉQUIPEMENT DE MAMMOGRAPHIE, PROCÉDÉ DE GÉNÉRATION D'IMAGES DE MAMMOGRAPHIE, ET ÉQUIPEMENT DE MAMMOGRAPHIE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:  **21.02.2018   IT 201800002904**

(43) Date of publication of application:
**05.02.2020   Bulletin 2020/06**

(73) Proprietor: **IMS Giotto S.p.A.**
**40037 Sasso Marconi (Bologna) (IT)**

(72) Inventors:
• **VECCHIO, Sara**
**40033 Casalecchio di Reno (BO) (IT)**

• **VIGNOLI, Paolo**
**40017 San Giovanni in Persiceto (BO) (IT)**

(74) Representative: **De Ros, Alberto et al**
**Studio Legale Bird & Bird**
**Via Borgogna, 8**
**20122 Milano (IT)**

(56) References cited:
**US-A1- 2007 114 424     US-A1- 2008 167 552**

• **Paolo Guerrieri: "Confronto di due software di monitoraggio della dose in mammografia: un'esperienza preliminare", , 1 January 2015 (2015-01-01), pages 1-80, XP055517115, Retrieved from the Internet: URL:https://core.ac.uk/download/pdf/796196 28.pdf [retrieved on 2018-10-19]**

**EP 3 600 049 B1**

**Description**

**FIELD OF THE INVENTION**

The present invention relates to a method for calibrating mammography equipment, a method for generating mammography images, and mammography equipment that implements these methods.

**BACKGROUND**

X-ray mammography equipment of the dual-energy type has been known for many years although it is not yet so much widespread. US2007114424 A1 may be considered to disclose a method for calibrating X-ray mammography equipment of the dual-energy type, through a plurality of calibration elements associated with predetermined iodine concentration.

In 2012, the Applicant submitted a patent application relating to equipment also adapted to dual-energy mammography which was published as EP2491863A1.

X-ray mammography equipment of the dual-energy type requires appropriate calibration so that the contrast of the images produced is truly improved compared to the contrast of the images produced by "single-energy" mammography equipment, i.e. traditional mammographs.

About ten years ago the company GE, which is also well known in the medical sector, proposed and used for its equipment an analytical calibration method that serves to determine the coefficients of the combination formula of the low-energy image and the high-energy image; the method is based on the "Monte Carlo" technique; this method is described in the article by S. Puong et al, entitled "Dual-energy contrast enhanced digital mammography using a new approach for breast tissue canceling" in "Medical Imaging 2007: Physics of Medical Imaging"; the authors argue that the measurements to be performed on phantoms with experimental calibration are "inconvenient".

The Applicant has realized that this analytical calibration method is of considerable computational complexity and therefore requires a suitable electronic computer (more powerful than the one integrated within mammography equipment) to perform the calculations in a reasonable amount of time.

Furthermore, the Applicant has realized that this analytical calibration method requires that the spectra of the X-ray beams emitted (as regards the low-energy beam and the high-energy beam) and the frequency response of the X-ray detector are known in advance. This also means that the calibration should be repeated whenever changes occur in the mammography equipment that modify the emission or transmission or reception of the X-rays, particularly when replacing a tube, a filter, a grille or a detector.

The general object of the present invention is to improve the known art. This object is achieved thanks to the calibration method, the imaging method, and the mammography equipment having the characteristics expressed in the appended claims which form an integral part of the present description. A first important element for the present invention is the use for calibration of a plurality of calibration elements containing iodine; in this way, the calibration is simplified because a low-energy image and a high-energy image are sufficient for each calibration element.

A second important element for the present invention is the use of a plurality of calibration elements associated with predetermined iodine concentration pairs in the element and glandularity configuration of the element for calibration; in this way, the calibration is very accurate, as is the imaging. As is known to those skilled in the art, the "glandularity" of a portion of an animal body (in particular human) refers to the weight or volume ratio of glandular tissue in the portion of the animal body and the entire portion of the animal body considered.

"Glandularity configuration" of a calibration element is intended (for example) as the glandularity value of a portion of an animal body having the same volume and the same absorption of X-rays.

A third important element for the present invention is the use of a plurality of calibration elements having different element thicknesses for calibration; in this way, the calibration is very accurate, as is the imaging.

A fourth important element for the present invention is the choice of a suitable combination formula and an appropriate regression technique which are a good compromise between the quality of the mammography image and the complexity of the calibration.

Preferably, the combination formula is a quadratic formula and therefore the coefficients to be determined through calibration are at most six.

Preferably, the regression technique corresponds to the least squares method.

**LIST OF FIGURES**

The present invention shall become more readily apparent from the detailed description that follows to be considered together with the accompanying drawings in which:

Fig. 1 shows a very schematic view of X-ray mammography equipment of the dual-energy type according to the present invention,

Fig. 2 shows a flow chart relating to three calibration methods according to the present invention, and

Fig. 3 shows a flow chart relating to three imaging methods according to the present invention.

As can be easily understood, there are various ways of practically implementing the present invention which is defined in its main advantageous aspects in the appended claims and is not limited either to the following detailed description or to the appended claims.

## DETAILED DESCRIPTION

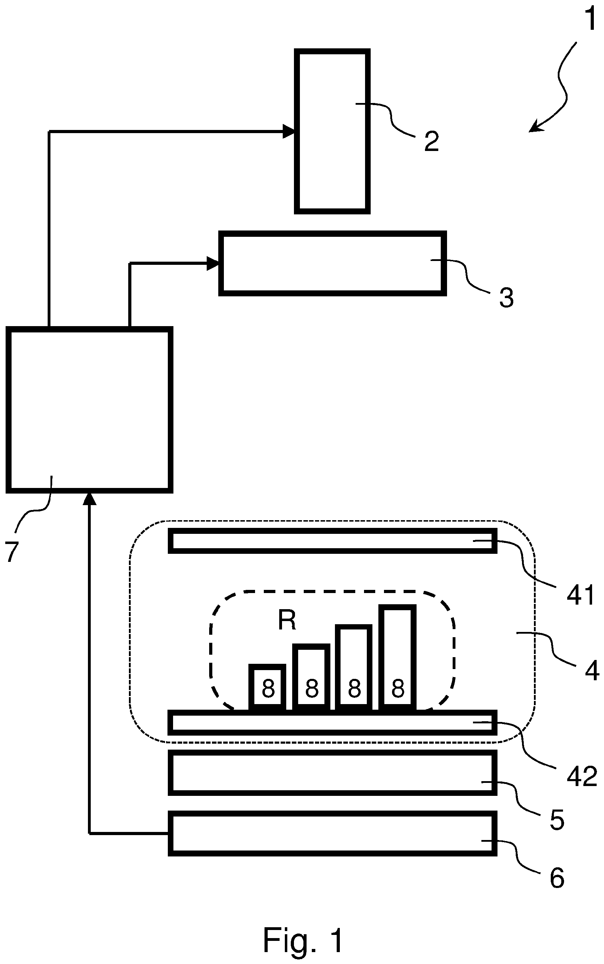

In Fig. 1, the numerical reference 1 comprehensively indicates the X-ray mammography equipment of the dual-energy type.

The equipment 1 comprises: at least one X-ray emitter 2, at least one corresponding filtering group 3, a breast positioning group 4, a grille group 5, an X-ray detector 6, and a control and processing unit 7 equipped with a user interface (for example, a keyboard and a screen).

The group 4 comprises a first contact element 41 (typically adapted to be positioned above a breast to be examined) for the breast and a second contact element 42 (typically adapted to be positioned below a breast to be examined) for the breast; the group 4 is associated with a spatial region R where a breast to be examined is positioned and which is reached and crossed by X-rays emitted by the emitter 2. The elements 41 and 42 are adapted to press a breast between them; typically the element 42 is fixed and the element 41 is movable to obtain such pressing.

The group 3 comprises at least two filters, adapted to filter the X-ray beam emitted by the emitter 2; in particular, in a first operating condition the beam is filtered by a first filter thus obtaining at the output a "low-energy beam" (and therefore "low-energy radiation" of the body to be examined), and in a second operating condition the beam is filtered by a second filter thus obtaining at the output a "high-energy beam" (and therefore "high-energy radiation" of the body to be examined).

The unit 7 is adapted, among other things, to control the emitter 2 and the group 3, as well as to receive images from the detector 6 and to process them.

To perform the calibration of the equipment 1, a plurality of calibration elements 8 are used; in Fig. 1, four calibration elements 8 of the plurality having four different thicknesses are shown by way of non-limiting example (as will be better understood below, the four calibration elements 8 can be associated with the same iodine concentration in the element and the same glandularity configuration of the element). For example and as shown in Fig. 1, during a calibration step, a group of calibration elements 8 is located in the region R, in particular the calibration elements 8 are resting on the contact element 42.

In Fig. 1, the calibration elements are shown as separate objects and spaced in a horizontal direction. Alternatively, they could be joined to form a single device (so-called "phantom").

In Fig. 1, the calibration elements are shown as single-piece objects, each having a certain thickness. Alternatively, a calibration element could consist of the overlapping of several parts in the vertical direction; for example, the first element 8 could be the overlapping of two parts, the second element 8 could be the overlapping of three parts, the third element 8 could be the overlapping of four parts, the fourth element 8 could be the overlapping of five parts.

Each calibration element 8 is associated with a predetermined iodine concentration pair in the element and glandularity configuration of the element. In other words, the calibration element contains a certain (and known) amount of iodine (preferably evenly distributed inside the volume of the element), and is therefore associated with a volumetric iodine concentration which corresponds to the ratio between the amount of iodine contained in the element and the volume of the element; moreover, the calibration element is made (preferably evenly) of a material or composition of materials which makes it look similar to breast tissue from the point of view of X-ray absorption and thus behaves as if it had a certain (and known) glandularity, i.e. it presents a certain "glandularity configuration".

In a first case different from that shown in Fig. 1, the calibration elements of the plurality resting simultaneously on the contact element of the equipment could all have the same thickness, but be associated with different iodine concentrations in the element and with the same glandularity configuration of the element.

In a second case different from that shown in Fig. 1, the calibration elements of the plurality resting simultaneously on the contact element of the equipment could all have the same thickness, but be associated with different glandularity configurations of the element and with the same iodine concentration in the element.

Obviously, there are many other possible cases.

The detector 6 is adapted to "capture" images; typically, an image captured by the detector 6 is supplied to the unit 7 as a matrix or array of pixels because, often nowadays, the detector consists of a matrix or array of microscopic sensors.

With the term "image value" at a point (or a small area) of the image, the response provided by a sensor (or by a small number of nearby sensors) of the detector or its processing can be understood.

Typically in the field of X-ray imaging, the image value is given by the following formula:

$$\ln \left( \frac{\int_k I_0(E) \cdot Q(E) \cdot dE}{\int_k I_0(E) \cdot e^{-\mu_a(E)t_a - \mu_b(E)t_b} \cdot Q(E) \cdot dE} \right)$$

which corresponds to the logarithm of the ratio between the response of a sensor in the absence of a body to be examined (deriving from a previous adjustment phase of the mammography equipment) and the response of the sensor in the presence of a body to be examined. From a physics perspective, this "image value" represents the attenuation, indicated with "D", that the X-rays have undergone along the path that goes from the X-ray emitter to a sensor of the X-ray detector due to the body under examination.

As is known, the image that X-ray mammography equipment of the dual-energy type presents to the user derives from a low-energy image capture, a high-energy image capture and a combination of the two images (more precisely, point by point image values) according to an appropriate pre-set combination formula in the mammography equipment. Often in the past, X-ray mammography equipment of the dual-energy type performed a "subtraction" (which is a simple form of linear combination) of two low and high-energy images; according to the present invention, a quadratic-type combination is preferred over the "subtraction", that is, a linear combination of two different "base materials" (see the formula on page 8) in turn calculated from the same pair of low and high-energy images according to the formula:

$$M_i = C_{0_i} + C_{1_i} D_{low} + C_{2_i} D_{high} + \\ + C_{3_i} D_{low}^2 + C_{4_i} D_{high}^2 + C_{5_i} D_{low} D_{high}$$

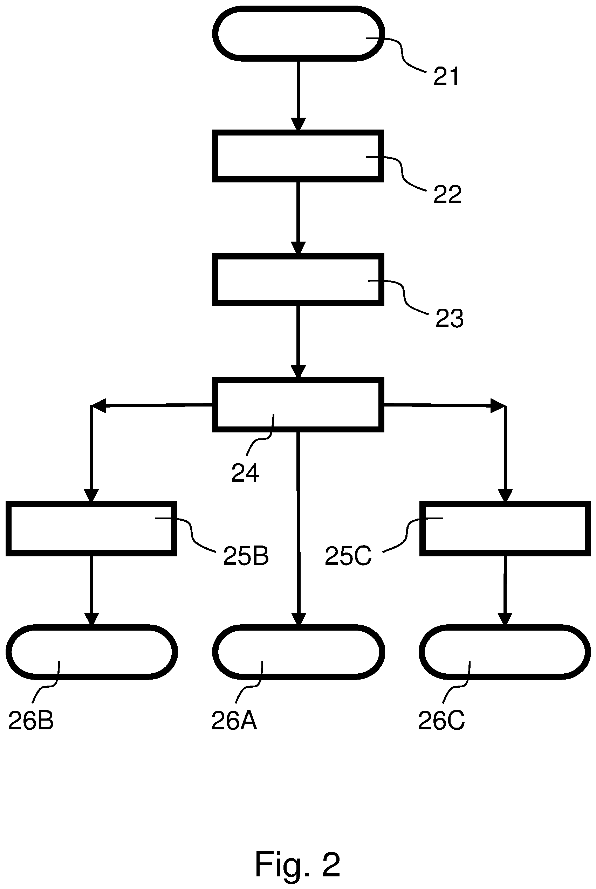

The calibration of the present invention serves to determine the coefficients of this combination formula and will be described below with the aid of Fig. 2 and Fig. 1; in the case of a quadratic formula, there are six coefficients to be determined, or less if any of the coefficients of the formula are assumed to be equal to zero, for each "base material".

As already said, in order to carry out an accurate calibration, according to the present invention a plurality of pairs of iodine and glandularity concentrations are taken into consideration; for example, it is possible to consider ten different iodine concentrations (or a different number comprised for example between five and fifteen) and four different glandularities (or a different number comprised for example between two and six) so that the different pairs are forty.

The calibration method according to the present invention comprises the steps of:

A) preparing (block 22 in Fig. 2) for each of said pairs a group of calibration elements having different and predetermined element thicknesses associated with the same pair, or more precisely associated with the same iodine concentration and the same glandularity configuration - for example, eight different thicknesses can be considered (or a different number comprised for example between four and twelve) and therefore the method requires 40x8=320 calibration elements (8 in Fig. 1),

B) for each calibration element obtain (block 23 in Fig. 2) from the mammography equipment (1 in Fig. 1) a low-energy image value through low-energy radiation and a high-energy image value through high-energy radiation, and

C) for each thickness calculate (block 24 in Fig. 2) the coefficients of the combination formula through a regression technique using the low-energy image values and the high-energy image values obtained in step B as well as the iodine concentration values of the calibration element subject to imaging from time to time.

As previously stated, "glandularity configuration" of a calibration element is intended (for example) as the glandularity value of a portion of an animal body having the same volume and the same X-ray absorption.

Note that a dual-energy imaging technique is based, inter alia, on the possibility of describing the X-ray behaviour of any material as a combination of the X-ray behaviour of two base materials according to the formula:

$$T = M_a \cos \Phi + M_b \sin \Phi$$

Typically, the contrast liquid used for this type of mammography examination contains iodine, and therefore performing calibration with calibration elements containing iodine not only means performing the calibration in more similar conditions to the actual examination, but also reducing calibration times and simplifying the calculations; in fact, in the formula above, Φ can be assumed as equal to zero and therefore a single set of coefficients can be calculated for each base material instead of a set.

In Fig. 2, an initial phase 21 is shown which is shared by the three methods shown in the figure.

A first method ends with the final step 26A and comprises only the three steps A, B, C indicated above. This means that, in this case, at the end of the calibration there are as many sets of coefficients as there are different thicknesses of calibration samples, for example eight sets of six coefficients. Typically, all these coefficients will be stored in the memory of the mammography equipment such that they can be used to create the combined image to be presented to the user, i.e. during normal use of the equipment.

It is best to clarify that mammography equipment is typically calibrated in relation to various parameters. For example, X-ray mammography equipment of the dual-energy type should be subjected to "flat-fielding" calibration (in particular specific "flat-fielding" calibration for dual-energy imaging) before being subjected to calibration of the "combination coefficients".

At the end of step B, low-energy image values are available for all the calibration elements, for example, 320 image values, and high-energy image values are available for all the calibration elements, for example, 320 image values.

In general, each image value will not derive from a distinct image capture. The image values of several calibration elements of the same thickness can be obtained by means of a single image capture, in particular all the calibration elements of the same thickness. For example, with a single capture, forty image values (low or high-energy) are obtained corresponding to forty pairs of iodine concentration and glandularity configuration; in this case, forty calibration elements 8 could be arranged in a matrix (for example four x ten) and rest on the contact element 42 in the region R, as shown in Fig. 1, and the resulting image could correspond to forty small squares or circles arranged in a matrix (for example four x ten).

Preferably, according to the present invention, the combination formula is a quadratic formula and there are six coefficients (for the construction of the image relating to iodine).

Preferably, according to the present invention, the regression technique corresponds to the least squares method.

During step B of the calibration, a plurality of image captures are usually carried out, in particular a plurality of pairs of image captures, by the mammography equipment. Preferably, in order for the calibration to be best performed, actions should be taken so that the signal/noise ratio of the images captured during the calibration is high; this is obtained by choosing a high value of the parameter mAs, in particular much higher (preferably 2-3 times higher) than that used to create the combined images to present to the user, that is, during normal use of the equipment.

Each of the images resulting from step B can contain a matrix of small squares or circles corresponding to calibration elements (8 in Fig. 1) resting on the lower contact element (42 in Fig. 1) of the group (4 in Fig. 1). If the calibration elements are manually placed on the contact element, the position of the matrix of small squares or circles can slightly vary from image to image. In order to obtain good alignment of the various images, the mammography equipment may request the intervention of an operator; the operator may be requested to indicate at least one image position (for example, the position of a vertex of the matrix can be indicated with a mouse); preferably, the operator can be requested to indicate two image positions (for example, the positions of two opposite vertices of the matrix can be indicated with a mouse) or an image position and an image angle.

A second method shown in Fig. 2 ends with the final step 26B and comprises, beyond steps A, B, C, also a step D (block 25B); in this step, for each of the coefficients of the combination formula, a "dotted linear" or "linear segments" interpolation function is calculated of the homologous coefficients obtained in step C relative to different thicknesses. This means that, in this case, at the end of the calibration there are as many linear functions available as there are coefficients, for example six for a quadratic combination. Typically, all these functions will be stored in the memory of the mammography equipment such that they can be used to create the combined image to be presented to the user, i.e. during normal use of the equipment.

A third method shown in Fig. 2 ends with the final step 26B and comprises, beyond steps A, B, C, also a step D (block 25C); in this step for each of the coefficients of the combination formula, a non-linear (for example cubic) interpolation function is calculated of the homologous coefficients obtained in step C relative to different thicknesses. This means that, in this case, at the end of the calibration there are as many non-linear functions available as there are coefficients, for example six for a quadratic combination. Typically, all these functions will be stored in the memory of the mammography equipment such that they can be used to create the combined image to be presented to the user, i. e. during normal use of the equipment.

Note that the calibration according to the present invention can be carried out while leaving the equipment in its normal place of use (for example in a hospital) and without having to bring it back to the manufacturer, provided that the calibration elements are available on site.

Moreover, the calibration according to the present invention requires operations and calculations which can be performed in little time (for example 15 minutes) and can therefore be repeated for example once a week or once a month.

Finally, the calibration according to the present invention requires calculations which can be carried out by the control and processing unit of the equipment and therefore does not require other equipment in addition to the calibration elements.

Matters of safety and/or liability may require that the calibration is carried out by the manufacturer and not by the user (i.e. the operator).

After the X-ray mammography equipment of the dual-energy type is completely and correctly calibrated, comprised the calibration according to the present invention, it is possible to start to use it, i.e. to carry out examinations on patients.

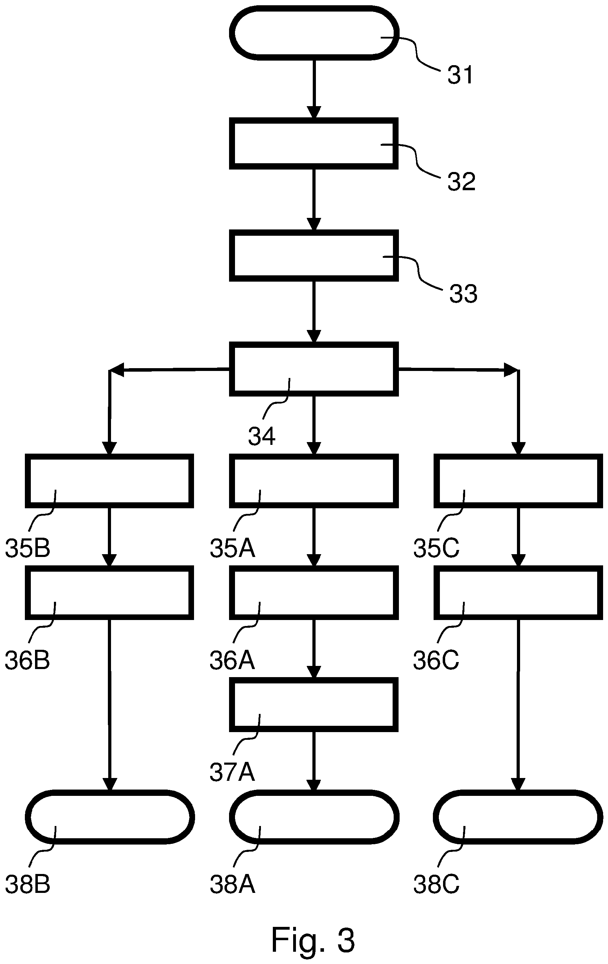

The imaging methods according to the present invention will be described below with the aid of Fig. 3 and Fig. 1.

With reference to Fig. 3, a first method for generating images using X-ray mammography equipment of the dual-energy type begins with the step corresponding to block 31 and ends with the step corresponding to block 38A.

With reference to Fig. 3, a second method for generating images using X-ray mammography equipment of the dual-energy type begins with the step corresponding to block 31 and ends with the step corresponding to block 38B.

With reference to Fig. 3, a third method for generating images using X-ray mammography equipment of the dual-energy type begins with the step corresponding to block 31 and ends with the step corresponding to block 38C.

In addition to the initial step 31, these three methods have steps 32, 33 and 34 in common.

In step 32, the mammography equipment determines the "real" thickness of the breast positioned in the region R of the group 4 between the element 41 and the element 42; this "real" thickness can be assimilated, for example, to the distance between the elements 41 and 42 after the breast has been completely pressed.

In step 33, the mammography equipment captures a low-energy image.

In step 34, the mammography equipment captures a high-energy image. Steps 33 and 34 could also be reversed.

According to the first method, in step 35A the set of coefficients corresponding to the thickness immediately below the "real" thickness is identified and the set of coefficients corresponding to the thickness immediately above the "real" thickness is identified, in step 36A the coefficients of the two previously identified sets are interpolated taking into account the difference between the "real" thickness and the immediately lower and upper thicknesses, in step 37A the low and high-energy images are combined using the coefficients thus interpolated, thereby generating the image to be presented to the user. Alternatively, in step 35A the set of coefficients corresponding to the thickness immediately below the "real" thickness is identified and the low and high-energy images are combined using this set of coefficients, in step 36A the set of coefficients corresponding to the thickness immediately above than the "real" thickness is identified and the low and high-energy images are combined using this set of coefficients, in step 37A these two combined images are interpolated, taking into account the difference between the "real" thickness and the immediately lower and upper thicknesses, thereby generating the image to present to the user. These two alternatives differ in that, according to the first alternative, the coefficients are linearly interpolated (less onerous from the computational point of view), while according to the second alternative the images are linearly interpolated.

According to the second method, in step 35B the combination coefficients are determined by calculating each of the linear functions stored during the calibration for the "real" thickness value, in step 36B the low and high-energy images are combined using the values of the coefficients previously determined, thereby generating the image to be presented to the user. According to the third method, in step 35C the combination coefficients are determined by calculating each of the non-linear functions stored during the calibration for the "real" thickness value, in step 36C the low and high-energy images are combined using the values of the coefficients previously determined, thereby generating the image to be presented to the user.

The calibration and/or imaging methods according to the present invention are advantageously used in X-ray mammography equipment of the dual-energy type.

Some embodiments of mammography equipment are adapted to be calibrated through a calibration method according to the present invention. In particular, this equipment comprises an electronic unit wherein one or more programs are stored which are adapted to carrying out at least steps B and C of the calibration method according to the present invention.

Some embodiments of mammography equipment are adapted to implement an imaging method according to the present invention. In particular, this equipment comprises an electronic unit wherein one or more programs are stored which are adapted to performing one of the previously described methods.

The calibration and/or imaging methods according to the present invention find advantageous use, for example, in the equipment described and claimed in the patent application which was published as EP2491863A.

The patent application published as EP2656789A1 describes and claims equipment that could also provide dual-energy imaging, although not explicitly stated. Thus the calibration and/or imaging methods according to the present invention find advantageous use, for example, also in this equipment.

Note that the calibration method according to the present invention can be used to calibrate not only mammography equipment, but also tomosynthesis equipment. Moreover, commercial equipment is often adapted to both mammography and tomosynthesis, and therefore the same calibration procedure (based on mammography "image values") according to the present invention could be valid both for subsequent mammography examinations and for the subsequent tomosynthesis examinations.

In some tomosynthesis equipment, the calibration for tomosynthesis could be performed as described above, but using the "image values" ("$D$") as the starting data obtained in a specific reconstruction plan (preferably at half-height).

## Claims

1. Method for calibrating X-ray mammography equipment (1) of the dual-energy type, through a plurality of calibration elements (8), each element being associated with a pair of a predetermined iodine concentration and glandularity configuration, comprising the steps of:

    A) providing (22) for each of said pairs a group of calibration elements having different and predetermined element thicknesses associated with the same pair,

    B) for each calibration element of said plurality, obtaining (23) from said mammography equipment (1) a low-energy image value through low-energy radiation and a high-energy image value through high-energy radiation, and

    C) for each thickness calculating (24) coefficients of a formula for combining the low-energy image value and high-energy image value, through a regression technique using the low-energy image values and high-energy image values obtained in step B and the iodine concentration values of said calibration elements; wherein the glandularity configuration is the glandularity value of a portion of an animal body having the same volume and the same absorption of X-rays; and the glandularity of a portion of an animal body refers to the weight or volume ratio of glandular tissue in the portion of the animal body and the entire portion of the animal body considered.

2. Method according to claim 1, wherein said combination formula is a quadratic formula and there are six of said coefficients, one or more of said coefficients being able to be equal to zero.

3. Method according to claim 1 or 2, wherein said regression technique corresponds to the least squares method.

4. Method according to any one of the preceding claims, wherein step B comprises a plurality of image capture pairs by the mammography equipment, and wherein for each pair an operator is requested to indicate at least one image position, preferably two image positions or one image position and one image angle.

5. Method according to any one of the preceding claims, wherein the image values of various calibration elements of the same thickness are obtained through a single image capture by the mammography equipment.

6. Method according to claim 5, wherein the low-energy image values of all the calibration elements of the same thickness are obtained through a single first image capture by the mammography equipment, and wherein the high-energy image values of all the calibration elements of the same thickness are obtained through a single second image capture by the mammography equipment.

7. Method according to any of the preceding claims from 1 to 6, comprising the further step of:
    D) for each of the coefficients of said combination formula calculating (25B) a linear interpolation function of homologous coefficients obtained in step C relative to different thicknesses.

8. Method according to any of the preceding claims from 1 to 6, comprising the further step of:
    E) for each of the coefficients of said combination formula calculating (25C) a non-linear interpolation function of homologous coefficients obtained in step C relative to different thicknesses.

9. Method for generating images through X-ray mammography equipment of the dual-energy type, wherein an image is obtained by combining (35A, 36A) a low-energy image and a high-energy image according to a combination formula and using (37A) the coefficients calculated in step C of the method according to any one of claims 1 to 6 previously carried out.

10. Method for generating images through X-ray mammography equipment of the dual-energy type, wherein an image is obtained by combining (36B) a low-energy image and a high-energy image according to a combination formula and using (35B) the linear interpolation functions calculated in step D of the method according to claim 7 previously carried out.

**11.** Method for generating images through X-ray mammography equipment of the dual-energy type, wherein an image is obtained by combining (36C) a low-energy image and a high-energy image according to a combination formula and using (35C) the non-linear interpolation functions calculated in step E of the method according to claim 8 previously carried out.

## Patentansprüche

**1.** Verfahren zum Kalibrieren von Röntgen-Mammographiegeräten (1) des Dual-Energie-Typs durch eine Vielzahl von Kalibrierungselementen (8), wobei jedes Element mit einem Paar einer vorbestimmten Jodkonzentration und Glandularitätskonfiguration verbunden ist, mit den folgenden Schritten:

A) Bereitstellen (22) einer Gruppe von Kalibrierelementen mit unterschiedlichen und vorbestimmten Elementdicken, die demselben Paar zugeordnet sind, für jedes der Paare,
B) für jedes Kalibrierungselement der Vielzahl das Erhalten (23) von dem Mammographiegerät (1) eines niederenergetischen Bildwerts durch niederenergetische Strahlung und eines hochenergetischen Bildwerst durch hochenergetische Strahlung,
und
C) für jede Dicke das Berechnen (24) von Koeffizienten einer Formel zum Kombinieren des niederenergetischen Bildwerts und des hochenergetischen Bildwerts durch eine Regressionstechnik unter Verwendung der in Schritt B erhaltenen niederenergetischen Bildwerte und hochenergetischen Bildwerte und der Jodkonzentrationswerte der Kalibrierungselemente; wobei die Glandularitätskonfiguration

der Glandularitätswert eines Teils eines Tierkörpers mit dem gleichen Volumen und der gleichen Absorption von Röntgenstrahlen ist; und die Glandularität eines Teils eines Tierkörpers bezieht sich auf das Gewichts- oder Volumenverhältnis von Drüsengewebe in dem betrachteten Teil des Tierkörpers und dem gesamten Teil des Tierkörpers.

**2.** Verfahren nach Anspruch 1, wobei die besagte Kombinationsformel eine Quadratformel ist und es sechs Koeffizienten gibt, wobei einer oder mehrere der Koeffizienten gleich Null sein können.

**3.** Verfahren nach Anspruch 1 oder 2, wobei die Regressionstechnik der Methode der niedrigstwertigen Quadrate entspricht.

**4.** Verfahren nach einem der vorstehenden Ansprüche, wobei Schritt B eine Vielzahl von Bildaufnahmepaaren durch die Mammographieausrüstung umfasst und wobei für jedes Paar ein Bediener aufgefordert wird, mindestens eine Bildposition anzugeben, vorzugsweise zwei Bildpositionen oder eine Bildposition und einen Bildwinkel.

**5.** Verfahren nach einem der vorstehenden Ansprüche, bei dem die Bildwerte verschiedener Kalibrierungselemente gleicher Dicke durch eine einzige Bildaufnahme durch das Mammographiegerät erhalten werden.

**6.** Verfahren nach Anspruch 5, wobei die Niedrigenergie-Bildwerte aller Kalibrierungselemente der gleichen Dicke durch eine einzige erste Bildaufnahme durch das Mammographiegerät erhalten werden, und wobei die Hochenergie-Bildwerte aller Kalibrierungselemente der gleichen Dicke durch eine einzige zweite Bildaufnahme durch das Mammographiegerät erhalten werden.

**7.** Verfahren gemäß einem der vorhergehenden Ansprüche von 1 bis 6, das folgenden weiteren Schritt beinhaltet:
D) für jeden der Koeffizienten der Kombinationsformel die Berechnung (25B) einer linearen Interpolationsfunktion von homologen Koeffizienten, die in Schritt C relativ zu verschiedenen Dicken erhalten wurden.

**8.** Verfahren gemäß einem der vorhergehenden Ansprüche von 1 bis 6, das folgenden weiteren Schritt beinhaltet:
E) für jeden der Koeffizienten der Kombinationsformel die Berechnung (25C) einer nichtlinearen Interpolationsfunktion von homologen Koeffizienten, die in Schritt C relativ zu verschiedenen Dicken erhalten wurden.

**9.** Verfahren zum Erzeugen von Bildern durch Röntgen-Mammographiegeräte des Dual-Energie-Typs, wobei ein Bild durch Kombinieren (35A, 36A) eines niederenergetischen Bildes und eines hochenergetischen Bildes gemäß einer Kombinationsformel und unter Verwendung (37A) der in Schritt C des Verfahrens berechneten Koeffizienten gemäß einem der zuvor ausgeführten Ansprüche 1 bis 6 erhalten wird.

10. Verfahren zum Erzeugen von Bildern durch Röntgen-Mammographiegeräte des Dual-Energie-Typs, wobei ein Bild durch Kombinieren (36B) eines niederenergetischen Bildes und eines hochenergetischen Bildes gemäß einer Kombinationsformel und unter Verwendung (35B) der linearen Interpolationsfunktionen, die in Schritt D des zuvor ausgeführten Verfahrens nach Anspruch 7 berechnet wurden, erhalten wird.

11. Verfahren zum Erzeugen von Bildern durch Röntgen-Mammographiegeräte des Dual-Energie-Typs, wobei ein Bild durch Kombinieren (36C) eines niederenergetischen Bildes und eines hochenergetischen Bildes gemäß einer Kombinationsformel und unter Verwendung (35C) der in Schritt E des zuvor ausgeführten Verfahrens nach Anspruch 8 berechneten nichtlinearen Interpolationsfunktionen erhalten wird.

**Revendications**

1. Procédé d'étalonnage d'un équipement de mammographie à rayons X (1) du type biphotonique, au moyen d'une pluralité d'éléments d'étalonnage (8), chaque élément étant associé avec une paire d'une concentration d'iode prédéterminée et une configuration de glandularité, comprenant les étapes suivantes :

   A) prévoir (22), pour chacune desdites paires, un groupe d'éléments d'étalonnage présentant des épaisseurs d'élément différentes et prédéterminées associées à la même paire,
   B) pour chaque élément d'étalonnage de ladite pluralité, obtenir (23) dudit équipement de mammographie (1) une valeur d'image à faible énergie au moyen d'un rayonnement à faible énergie et une valeur d'image à haute énergie au moyen d'un rayonnement à haute énergie,
   et
   C) pour chaque épaisseur, calculer (24) des coefficients d'une formule de combinaison de la valeur d'image à faible énergie et la valeur d'image à haute énergie, au moyen d'une technique de régression utilisant les valeurs d'image à faible énergie et les valeurs d'image à haute énergie obtenues à l'étape B et les valeurs de concentration d'iode desdits éléments d'étalonnage ; dans lequel la configuration de glandularité est

   la valeur de glandularité d'une partie d'un organisme animal présentant le même volume et la même absorption des rayons X ; et la glandularité d'une partie d'un organisme animal désigne le rapport de poids ou de volume entre le tissu glandulaire dans la partie de l'organisme animal et la totalité de la partie de l'organisme animal en question.

2. Procédé selon la revendication 1, dans lequel ladite formule de combinaison est une formule quadratique et lesdits coefficients sont au nombre de six, un ou plusieurs desdits coefficients pouvant être égaux à zéro.

3. Procédé selon la revendication 1 ou 2, dans lequel ladite technique de régression correspond à la méthode des moindres carrés.

4. Procédé selon l'une quelconque des revendications précédentes, dans lequel l'étape B comprend une pluralité de paires de capture d'image au moyen de l'équipement de mammographie, et dans lequel, pour chaque paire, il est demandé à un opérateur d'indiquer au moins une position d'image, de préférence deux positions d'image ou une position d'image et un angle d'image.

5. Procédé selon l'une quelconque des revendications précédentes, dans lequel les valeurs d'image de divers éléments d'étalonnage de même épaisseur sont obtenues par une seule capture d'image au moyen de l'équipement de mammographie.

6. Procédé selon la revendication 5, dans lequel les valeurs d'image à faible énergie de tous les éléments d'étalonnage de même épaisseur sont obtenues par une seule première capture d'image au moyen de l'équipement de mammographie, et dans lequel les valeurs d'image à haute énergie de tous les éléments d'étalonnage de même épaisseur sont obtenues par une seule seconde capture d'image au moyen de l'équipement de mammographie.

7. Procédé selon l'une quelconque des revendications précédentes 1 à 6, comprenant l'étape supplémentaire suivante :
   D) pour chacun des coefficients de ladite formule de combinaison, calculer (25B) une fonction d'interpolation linéaire de coefficients homologues obtenus à l'étape C par rapport à différentes épaisseurs.

8. Procédé selon l'une quelconque des revendications précédentes 1 à 6, comprenant l'étape supplémentaire suivante :
   E) pour chacun des coefficients de ladite formule de combinaison, calculer (25C) une fonction d'interpolation non linéaire de coefficients homologues obtenus à l'étape C par rapport à différentes épaisseurs.

9.  Procédé de génération d'images au moyen d'un équipement de mammographie à rayons X du type biphotonique, dans lequel une image est obtenue en combinant (35A, 36A) une image à faible énergie et une image à haute énergie selon une formule de combinaison et en utilisant (37A) les coefficients calculés à l'étape C du procédé selon l'une quelconque des revendications 1 à 6 exécuté précédemment.

10. Procédé de génération d'images au moyen d'un équipement de mammographie à rayons X du type biphotonique, dans lequel une image est obtenue en combinant (36B) une image à faible énergie et une image à haute énergie selon une formule de combinaison et en utilisant (35B) les fonctions d'interpolation linéaires calculées à l'étape D du procédé selon la revendication 7 exécuté précédemment.

11. Procédé de génération d'images au moyen d'un équipement de mammographie à rayons X du type biphotonique, dans lequel une image est obtenue en combinant (36C) une image à faible énergie et une image à haute énergie selon une formule de combinaison et en utilisant (35C) les fonctions d'interpolation non linéaires calculées à l'étape E du procédé selon la revendication 8 exécuté précédemment.

Fig. 1

Fig. 2

Fig. 3

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2007114424 A1
- EP 2491863 A1
- EP 2491863 A
- EP 2656789 A1

**Non-patent literature cited in the description**

- **S. PUONG et al.** Dual-energy contrast enhanced digital mammography using a new approach for breast tissue canceling. *Medical Imaging 2007: Physics of Medical Imaging,* 2007